# EUROPEAN PATENT APPLICATION

(11) **EP 1 284 291 A1**
(43) Date of publication of application: **19.02.2003**
(21) Application number: 01934335.9
(22) Date of filing: 24.05.2001
(51) Int. Cl.: C12N 15/12, C12N 5/10, G01N 33/15, G01N 33/50

(54) **HUMAN PGC-1 PROMOTER**

(30) Priority: 25.05.2000 JP 2000155098
(71) Applicant: YAMANOUCHI PHARMACEUTICAL CO. LTD., Tokyo 103-8411 (JP)
(72) Inventor: GOTO, Masahide, Tsukuba-shi, Ibaraki 305-8585 (JP); SHIMOKAWA, Teruhiko, Tsukuba-shi, Ibaraki 305-8585 (JP)
(74) Representative: HOFFMANN - EITLE
(86) International application number: JP0104361
(87) International publication number: WO01090356

(57) **Abstract**

A DNA having an hPGC-1 promoter activity; a method of screening a substance modifying this promoter activity; and drugs, which contain substances modifying the promoter activity as the active ingredient, for activating mitochondria and treating obesity and/or diabetes based on the activation of mitochondria.

SEQUENCE LISTING

<110> Yamanouchi Pharmaceutical Co., Ltd.

<120> Human PGC-1 Promoter

<130> Y0114-PCT

<140>
<141>

<150> JP 2000-155098
<151> 2000-05-25

<160> 7

<170> PatentIn Ver. 2.1

<210> 1
<211> 5235
<212> DNA
<213> Homo sapiens

<220>
<221> promoter
<222> (1)..(5235)

<400> 1
actatagggc acgcgtggtc gacggcccgg gctggtactc aagcactaaa aaataagtat 60

cacgggaatg gtttgattgc tttttttttt ttttttcagt taaacaattt gtgtacctta 120
attttccctg gcacttacta agttcagcta gtgtgaaaaa aaaaatcctt cttagtaaat 180
gaaatgttat gttatgtatt gaagttctgt agcactttaa aattttaaat agtgccaaat 240
ttgacaatat gtaatatgcc ttgttcagtt ccacttaaaa aaattgctag ataatcctat 300
tgaacattta ttgtctaaat aatcaataac tgtctgcatt atagataaat taacctggaa 360
taattaaggt aaatgaaaat gtggattcac aaatacaacc gagttgaaca tcagacaaaa 420
tcagatttta accacccttt tattatctct tatctctgtg cttaataagt acaattgttg 480
ctttaagttt agattagtcc aattgactgc aactcagatt aactttcatg ttattttttg 540
ttttaatatg aaaaacacca aaagcacatt ttgaggtcca aattaaaata gaggctgact 600
cagttccata gagccggtct gcagaaggtt ttactggaga tgagagatgt ttttgctaag 660
gttgtctatc ataacttgtt tattaaaaaa ataaaaataa atgctaggag acattcaagc 720
tgtcttgatt ttcctctagt tatttcctca aaggttcttt gttcacatgg acatcactgt 780
ggcatttttt tcaagcaaaa tgaggtttaa ttttatagta tttatagaat agaagtcttt 840
gttatatcct atcctgctac caacttttgt gttctatgca gtctctgctt ctaaccagag 900
gcccaaagca tgtgtttaga aactgatgtg gggtatcagg atcaattagt ccctctagga 960
attaaaagca agcaattgaa tgcctgttaa caatgactaa cttgaaaaat attagcaatt 1020
ctaggcaggc ccctttgctt tctggacgaa tgctctaggc aggcagcatt cccaggaata 1080
ttggtttcca gctatgtaga ctgtccctca gttataaagt tgcctccagg gtctttgcta 1140
tggctttgat ttgaggatga ttattattct tatggatgaa actttagttc ttcctagaaa 1200
ggctttcagc gatttgcatg ctctttccag ctgccctcca aaccttgccc agccttattt 1260
ttctccatca taaaatgggt acacaggtca tttttattgg atagtttgga ggactaaatt 1320
gattgagatg tggtatatga agtgctgatc ttaatgtcta gcccatacgt ggtctgctgt 1380
atatgtcttg ctttccacct ttctttcttg agaagtttct gcatacatat attcttatgt 1440
agttattgta taaatagtgt atatggaaac attcccgagg ttgtattttc ctgttctttc 1500
ttgatttaga aaactaaaac tacagtaatt ttctgctgtg tgtaagagag aaaatgtgct 1560
tatgaactgt attcagcaaa cttaaaatgt taaagctctt ggtcaccttt ttatgcccag 1620
taatgataaa ctgtcaattt gcttacgtta tttgggaatt tactagccac ttgctgcaga 1680
tatcacagta gacacatctc ttataatatg tatgatctga aagaagcagc agtctggtag 1740
tttttgtttt gttttattaa aataaaaact tataaactta taaaaacaaa tgaacaaata 1800

tatttatcct aatagctctt ctgagtttgc ttctgaaatc acctatattc aatatagaca 1860
acaaaaccaa ttgtttttgc tattctctga ggtttttgag tttgagggtt tgaatttgat 1920
tctaattttt agaaatgggc agttgtctga aatatggagg gatgggaaaa gcctttaatt 1980
tggagtagga gtgaggaagg aaacccaaat gttatacact tcagtcaacg tatttgttag 2040
agaattgaat tattctcttg aggttttttt ttttctcttt gtctgcaact cgagatagat 2100
tctcctgcct ctactaatat tgaaaccctg gaaaatgtta acagaactac agaggggaga 2160
aattatcagg atggaatgta ggctttgtgt agatatgcaa cattgggggc actcagcaga 2220
tgttaataaa tacctctttc tattgttgag aaaaaatggg tttgatttgg gtgtgaactg 2280
cagtggtcaa tgaaaggaac ttgtatctca ccagggtgtg ttttaagcag tagaagagga 2340
atctgggtgt tgatccaagt gacgttggtt tttcatacca aattattccc catttttaag 2400
ggttgtcatc cccctaaaat tgtaagctct cttgtgtcca tgtcatatgc tttcttggca 2460
gtccttgtga tacatggctg tgcacacagt aaggtttcaa tagatccttg ttgtctgaaa 2520
ccagatattt gggaattttg gagacagagt tgacgaaggg gtgaaaggag ggctgtgggt 2580
agagagtaag agaaatagtc aaagcagaga gagaattaaa tagagaaatg gcccggggaa 2640
ccagaaaaaa accaaggttg gtgagcaatg aaccatttaa gttctcctga gaaaagaaaa 2700
aaaaaaaact ggactgaaga aaaaatttaa tctgaagagt taattaccaa aattataaaa 2760
cctctaaaac atccctctca ttacagttgt agttttaaga caaaaaaaaa aaaatatcgt 2820
ggaaagcact ctaatttgtc ctaggaatct atttaatttt gatttgtcca gtaatagaaa 2880
tttgaggggg aaatgcaata tactgtagtt tatgagtctt ttaaatgttg ttacttctga 2940
attccatcct ctttgtgata gtgagagtga agaaaacaca agtgtatttg aaatcacttc 3000
tggtttcagt gcatgtgctg atttgatttt ttagaagtag aattagatgg caaaaaataa 3060
gaaaacccta acaatacaat ctgggcaggt tagaatatca aaaattataa cgagcctgtt 3120
ctaatgtaaa gaacatctct aaaagatgtg aataggtttt aaaaatatga tgaatatcta 3180
gcattggata aacacaaata aaggtatcag tgaatatcac aatggaagaa tcacatgata 3240
ataattaata aacattgact cttcttggcc aggtgaccaa tattatttca cagttgatcg 3300
ttgagggcag agccaatgac ctttgtcctg aattttaata atttactgaa gttttacatt 3360
aaatcagaat ataattggga agaaaattag tgtttctttg tggggagtgc agagtaatgc 3420
cactaattgc ttgtataaaa gcattcccga ggttgtattt tcctgttctt tcattagaaa 3480
attaaaacta cagtaatttt ctgctgtttg taaaagggaa aatgtgctgg tgaactgtat 3540

tcagcaaact taaaatgtca aagctcttgg ccaccttttt atgcccagta atgataaact 3600
gtcagcttcc ttacattatt tgggaattta ctagcaactt gttgcagctc tcacagcaca 3660
caaatctctt ataatatgta tggtctgaaa gaagcagcag tctgctggta ttttgtttct 3720
tttgttcttt ctttcacttt aatatttgag caatttaata tttaaaatat gtgttagggc 3780
aaataaccat gtttttctgt ttaaggagat ggacaatgaa gaacagtgta aattaacctc 3840
agtagaataa tgctttacaa attatattta ggtaactggg ggattgtttt caggtaaatt 3900
attatcaagt aaaagaattg agctgattct aaaagtatat tgggtttaac attttatgaa 3960
aacatgttta ttcacacaga tacatacacg tgtacatctc ctgggaactt ggaagggtta 4020
agtcagacca ctgtcatgtt acaggaagtg tttttccact gtgtccagta ccttgagttt 4080
gttatgtatt caatatgtag tgtcatcata aaacagttgc acctacctgc attagccctc 4140
attgtctcaa ggtacaagct gaaaaataat agaaagtaaa gctcaggaat tgaatatttc 4200
tgctaatagt gtgttggtat ttttccctca gttcacagac attcttgatt tcaaaacgca 4260
aactacacaa cccagggcac tagggttgga attcaatgtt tattcaaaaa ggcaccctaa 4320
ggcagttagg gaggaaacgc tacatgtatg aaaaatagga gccgggaatc aaagctgatc 4380
tgagcagagc agcagcgact gtatttacta acacttgttt tctgggagcc tatgagagaa 4440
atggaaataa ttagaaggaa gctgaaagga tggggttttg tggcttgttc tccttatatg 4500
gagcaaagaa aactgcagca actcttcggg agctggtatt ccctactgcc atgggggcag 4560
ccgaattctg ggtggaggag tttgtttata ccttaacaca tacaggctat tttgttgatt 4620
aaacaagcaa aaaaaaaaaa aaaagccccg tttgcgcttt caaacactcc ctcaatgaga 4680
aaatgtctca taaaaatgca tcatgtgata agctcttgct ttagtcccaa actgagcttg 4740
agtccacttg gagatcttag aattaaagag ttcttaggga atacacgttt tagctaagaa 4800
tatagttact ctgtcatgaa acagggagct ttgccacttg cttgttttgg aaggaaaata 4860
aattaaaaaa agattgcagg ggattttggt tattatatgg ccagggctcc gtttagagtc 4920
tgtggcattc aaagctggct ttaatcacag catgatgctt gaagcctcca aaagtctaag 4980
tgtttccttt ctttctttct tttctttttc tttctttttt tttttaaagc gttacttcac 5040
tgaagcagag ggctgccttt gagtgacgtc acgagttaga gcagcaagct gcacagggga 5100
agggaggctg ggtgagtgac agcccagcct actttttaat agctttgtca tgtgactggg 5160
gactgtagta agacaggtgc cttcagttca ctctcagtaa ggggctggtt gcctgcatga 5220
gtgtgtgctc tgtgt 5235

<210> 2

<211> 28

<212> DNA

<213> Artificial Sequence

<220>

<223> Description of Artificial Sequence:an artificially

synthesized primer sequence

<400> 2

atccagctcc tgaatgacgc cagtcaag 28

<210> 3

<211> 31

<212> DNA

<213> Artificial Sequence

<220>

<223> Description of Artificial Sequence:an artificially

synthesized primer sequence

<400> 3

acacagatct cacactcatg caggcaacca g 31

<210> 4

<211> 30
<212> DNA
<213> Artificial Sequence

<220>
<223> Description of Artificial Sequence:an artificially
      synthesized primer sequence

<400> 4
aacccagggc acgcgtgttg gaattcaatg                                    30

<210> 5
<211> 30
<212> DNA
<213> Artificial Sequence

<220>
<223> Description of Artificial Sequence:an artificially
      synthesized primer sequence

<400> 5
agggaatacg cgttttagct aagaatatag                                    30

<210> 6
<211> 30
<212> DNA
<213> Artificial Sequence

<220>

<223> Des.cription of Artificial Sequence:an artificially

synthesized primer sequence

<400> 6

tttaacgcgt tacttcactg aagcagaggg 30

<210> 7

<211> 31

<212> DNA

<213> Artificial Sequence

<220>

<223> Description of Artificial Sequence:an artificially

synthesized primer sequence

<400> 7

ctacttttta aacgcgttgt catgtgactg g 31

## Description

### Technical Field

The present invention relates to a DNA having a promoter activity of human PGC-1 (hPGC-1) gene, a screening method using the DNA, and a pharmaceutical composition having an activity to activate mitochondria, which comprises as an active ingredient a substance stimulating the promoter activity.

### Background Art

"Obesity" is one of life-style related diseases induced by unbalance of energy intake and its consumption, and it is understood that "excessive accumulation of fat" occurs as a result of excessive intake of energy or shortage of exercise. Also, the obesity is medically understood as a risk factor which enhances incidence rate of "life-style related diseases" such as non-insulin dependent diabetes mellitus (NIDDM), arteriosclerosis and certain cancers, and an effective treating method therefor is requested (Diabetes Journal, 27(3), 1 - 6 (1999)). In particular, with regard to obesity and diabetes (especially, non-insulin dependent diabetes mellitus), a causal relationship between them is suggested as follows (Diabetes journal, 27(3), 87 - 92 (1999)). Spiegelman et al. have proposed "hypothesis of functional inhibition of insulin receptor" that insulin resistance is characterized by inhibition of insulin receptor function caused by a cytokine (TNF-α) secreted from enlarged adipocytes (Science, 259, 87 - 91 (1993)). Matsuzawa et al. have further proposed "adipocentric hypothesis" which includes understanding that insulin resistance in skeletal muscles, liver and pancreatic β cells is induced by increase of adipose tissues owing to obesity and related increase of secretion of various resistance-inducing factors (Diabetes Journal, 27(3), 87 - 92 (1999)). Moreover, Unger et al. have suggested that fat accumulated in liver, muscles and pancreatic β cells can be a cause of insulin resistance (Diabetes, 44, 863 - 870 (1995); Am. J. Physiol., 273 (Endocrinol. Metab. 36): E708 - E713 (1997); Metabolism, 49(1), 108 - 114 (2000)).

With the recent advance of molecular genetics and molecular biology, from the analysis of hereditary obesity model mouse/rat and human family based on single-gene abnormality, some genes taking part in the onset of obesity have been reported (Science, 280, 1363 - 1390 (1998)). For example, hitherto identified are leptin, β 3 adrenergic receptor, peroxisome proliferator-activated receptor γ, (PPAR γ), proopiomelanocortin (POMC), melanocortin-4 receptor (MC4R), and carboxipeptidase E (CPE), and the like (Chiryo-gaku (Therapeutics), 33(12), 60 - 66 (1999)). Research and development for creating drugs on some of the molecules mentioned above have been advanced for the purpose of treating obesity and non-insulin dependent diabetes mellitus. Among them, with regard to leptin, it is reported that it acts on hypothalamus to reduce appetite and also exhibits actions to raise body temperature and to enhance energy production at the periphery, and body weight loss by intravenous administration at a low dose without adverse effect was verified in a clinical test of human phase I (Nikkei Biotechnology Annual Report, p245, (1998)). However, with regard to the possibility of leptin as an antiobesity drug, problems to be solved in future still remain because a large species difference or personal difference is observed, data suggesting the existence of leptin-resistant patients are obtained, and it has a defect common to protein preparations (Nikkei Bio Yearbook, 99, 96 - 97 (1999); Nature 2000, 404: 538 - 540). In addition, with regard to β 3 adrenergic receptor, search for its agonist has been carried out at preclinical and clinical levels, and it acts especially on visceral fat to cause increase of heat production and as a result an antiobesity effect is expected. However, the compounds obtained in present contain problems in view of adverse effects, e.g., increase of mobilization level of free fatty acids in blood, actions on heart such as increase of heart rate, tremor, and the like, and thus no satisfactory medical agent has been developed yet (Science, 280, 1383 - 1387 (1998)).

As antiobesity drugs already commercialized, there are known those having a mechanism of central action. For example, the use of "sibutramin", a serotonin-reuptake inhibitor was permitted in U. S. A. in 1988 only for the purpose of preventing "excessive eating" attributed to mental stress. In Japan, as an appetite center depressant, the use of mazindol is permitted under a condition limited to severe obesity of BMI = 35. However, according to the evaluation of antiobesity action based on FDA evaluation standard, there is a report that no drug efficacy is observed in both compounds and thus they are not sufficiently satisfactory in view of efficiency (Endocrine Reviews, 20(6), 805 - 875 (1999)).

As mentioned above, no sufficiently satisfactory medical agent for obesity and non-insulin dependent diabetes mellitus attributed thereto is not yet found.

In addition to the obesity-related factors found as single-gene abnormality mentioned above, several genes having a possibility of causing obesity from the researches on mechanism analysis have been found. For example, it is reported that Uncoupling Protein 1 (UCP1) causes heat production in mitochondria by its uncoupling. In animals wherein UCP1 specifically expressed in brown adipose tissue is inhibited, it is known that the increase of body weight is induced (Nature, 366, 740 - 742 (1993)). Contrary, it is known that obesity resistance is exhibited when UCP1 is excessively expressed in mouse (J. Clin. Invest. 96, 2914 - 2923 (1995)).

Recently, as a new factor taking part in the heat production, PPAR γ coactivator-1 (PGC-1) derived from mouse was found (Cell, 92, 829 - 838 (1998)). The gene interacts with nuclear receptor PPAR γ and cloned as a factor assisting its transcriptional activity. It is hitherto reported that PGC-1 enhances the expression of UCP1 gene controlled by PPAR γ to a large extent. Moreover, PGC-1 includes the expression of NRF (Nuclear Respiratory Factor) which is one of transcription factors working in mitochondria and UCP2 which is one of subtypes of UCP1 and considered to cause energy consumption in mitochondria (Cell, 98, 115 - 124 (1999)). Furthermore, it was found that forced expression of PGC-1 by gene transfer induces the expression of a transcription factor mtTFA (mitochondrial transcription factor A) playing an important role at genome duplication and transcription reaction in mitochondria, increases oxygen consumption in cells and increases number of mitochondria in a cell (Cell, 98, 115 - 124 (1999)). Furthermore, it was also reported after the priority date of the present application that PGC-1 induces the expression of a glucose transport protein GLUT4 in muscle cells and increases glucose transportation (PNAS, 98, 3820 - 3825 (2001)). However, there is no report on a novel antiobesity drug or antidiabetes drug which has a mechanism of PGC-1 expression enhancing action or its function enhancing action.

Moreover, WO 00/32215 opened to public after the priority date of the present invention first discloses the sequence of hPGC-1 coding region but any DNA having a PGC-1 promoter activity derived from any species is not known yet and there exists no assay system suitable for screening a substance stimulating an hPGC-1 promoter activity and enhancing the expression of hPGC-1.

### Disclosure of the Invention

It is an object of the invention to provide a DNA having an hPGC-1 promoter activity (hereinafter, promoter DNA) which is useful for screening a substance stimulating an hPGC-1 promoter activity and enhancing the expression of hPGC-1. Furthermore, it is another object to provide a screening method for a substance stimulating an hPGC-1 promoter activity using the DNA, and a medicament, particularly antiobesity and antidiabetes drugs having an action mechanism of activating mitochondria, the medicament containing as an active ingredient a substance stimulating the promoter activity.

As a result of the intensive studies for solving the above problems, the present inventors have succeeded in the isolation of a region having a promoter activity composed of about 5.2 kb, upstream of hPGC-1 gene, from human genome DNA. Also, they have identified a region of about 200 b which is essential for exhibiting a promoter activity in the promoter region composed of about 5.2 kb. Moreover, they have established a screening method for a substance controlling the promoter activity by the use of the isolated promoter DNA. Furthermore, by obtaining a substance stimulating the promoter activity according to the screening method and verifying that the substance activates mitochondria in muscle cells, they have found that the substance exhibits antiobesity and antidiabetes effects and thus accomplished the invention.

Namely, the invention relates to:
(1) A DNA having a human PGC-1 promoter activity, which comprises at least the base sequence of from the base number 5023 to the base number 5235 in the sequence of SEQ ID NO:1 and is a base sequence corresponding to all or part of the sequence of SEQ ID NO:1;
(2) The DNA as described in (1), which is selected from the group consisting of the base sequence of from the base number 5023 to the base number 5235 in the sequence of SEQ ID NO:1, the base sequence of from the base number 4776 to the base number 5235 in the sequence of SEQ ID NO:1, the base sequence of from the base number 4269 to the base number 5235 in the sequence of SEQ ID NO:1, the base sequence of from the base number 2816 to the base number 5235 in the sequence of SEQ ID NO:1, and the base sequence of from the base number 1 to the base number 5235 in the sequence of SEQ ID NO:1;
(3) A recombinant vector comprising the DNA as described in (1);
(4) A transformant comprising the DNA as described in (1);
(5) A screening method for a substance controlling the human PGC-1 promoter activity of the DNA as described in (1), which comprises a step of bringing a test substance into contact with the DNA and a step of measuring the human PGC-1 promoter activity;
(6) The screening method as described in (5), wherein the DNA as described in (1) has a reporter gene in the downstream and the step of measuring the human PGC-1 promoter activity is a step of detecting expression of the reporter gene;
(7) The screening method as described in (5), which aims at selection of a mitochondria-activating substance;
(8) The screening method as described in (7), wherein the mitochondria-activating substance is a substance for treating or preventing obesity and/or diabetes;
(9) A substance for controlling a human PGC-1 promoter activity, which is capable of being selected by the method as described in (5);
(10) A pharmaceutical composition for activating mitochondria comprising as an active ingredient a substance stimulating a human PGC-1 promoter activity, which is capable of being selected by the method as described in (5);
(11) The pharmaceutical composition as described in (10), which is a pharmaceutical composition for treating or preventing obesity and/or diabetes;
(12) A process for producing a pharmaceutical composition for activating mitochondria, which comprises a step of screening a substance stimulating a human PGC-1 promoter activity by the method as described in (5) and a step of preparing a formulation using the substance stimulating the promoter activity; and
(13) The production process as described in (12), wherein the pharmaceutical composition for activating mitochondria is a pharmaceutical composition for treating or preventing obesity and/or diabetes.

In this connection, the base sequence of from the base number 5023 to the base number 5235 in the sequence of SEQ ID NO:1 corresponds to from -189 to +23 of hPGC-1 gene, the base sequence of from the base number 4776 to the base number 5235 in the sequence of SEQ ID NO:1 to from -436 to +23 of hPGC-1 gene, the base sequence of from the base number 4269 to the base number 5235 in the sequence of SEQ ID NO:1 to from -943 to +23 of hPGC-1 gene, the base sequence of from the base number 2816 to the base number 5235 in the sequence of SEQ ID NO:1 to from -2396 to +23 of hPGC-1 gene, and the base sequence of from the base number 1 to the base number 5235 in the sequence of SEQ ID NO:1 to from -5211 to +23 of hPGC-1 gene.

Among the screening methods for a substance controlling an hPGC-1 promoter activity according to the invention, particularly preferred is the screening method for a substance stimulating an hPGC-1 promoter activity and, as a "reporter gene", preferred is the use of luciferase gene. The "expression of a reporter gene" can be detected by detecting the activity of the polypeptide (reporter activity) coded by the gene.

Among the substances for controlling an hPGC-1 promoter activity according to the invention, particularly preferred is a substance for stimulating an hPGC-1 promoter activity. Also, it is particularly preferred that the pharmaceutical composition for treating or preventing diabetes is a pharmaceutical composition for treating or preventing non-insulin dependent diabetes mellitus.

Moreover, the "activation of mitochondria" herein means the increase of mitochondrial enzymatic activity in cells and includes both of the case of increased activity per mitochondrion and the case of increased number of mitochondria in a cell, and "capable of being selected" includes both of the case of "actually selected by the screening method of the present invention" and the case of "can be selected".

The following will describe the invention in detail.

### 1) The DNA's, recombinant vectors, and transformants of the invention

The present inventors have synthesized a primer having a complementary sequence of mouse gene based on the gene information of PGC-1 coding region derived from mouse (GenBank Accession No. AF049330) and obtained a partial sequence of the upper stream of hPGC-1 gene using human genome library contained in the kit described in Examples. Thereafter, a sequence has been designed based on the resulting hPGC-1 gene sequence and an upstream sequence of about 5.2 kb containing hPGC-1 promoter region has been obtained by 5'-RACE method using sequentially an antisense nucleotide composed of 28 bases (SEQ ID NO:2) or 31 bases (SEQ ID NO:3) of 5'-terminal region of hPGC-1 gene, which is chemically synthesized, and then the base sequence of the upstream sequence has been determined (SEQ ID NO:1). The full length of the DNA fragment has been subcloned into an appropriate plasmid, specifically a plasmid pGVB-2 containing luciferase gene as a reporter gene. By detecting the expression of reporter gene of the fusion plasmid, i.e., the expression of luciferase by the activity, it has been verified that the resulting upstream DNA region has a promoter activity. Also, the inventors have found a minimum region composed of about 200 b responding to retinoic acid in the base sequence described in SEQ ID NO:1 and having an hPGC-1 promoter activity. In order to screen a substance controlling an hPGC-1 promoter activity, it is necessary to use at least the minimum region in the assay. Moreover, since the screening of a substance controlling an hPGC-1 promoter activity has been enabled by the use of a DNA containing the minimum region instead of the full length of about 5.2 kb, the search for a substance controlling an hPGC-1 promoter activity has been facilitated.

The hPGC-1 promoter DNA of the invention contains at least the base sequence of from the base number 5023 to the base number 5235 in the sequence of SEQ ID NO:1 (the portion corresponding to from -189 to +23 of hPGC-1 gene) and may further contain any base sequence of the 5' region of hPGC-1 gene described in SEQ ID NO:1.

The DNA of the invention can be also produced by the following processes in addition to the process described in Examples.

### (1) Production using the PCR method

A DNA containing the base sequence from the base number 5023 to the base number 5235 can be produced by synthesizing a primer composed of the base sequence corresponding to the 5' side from the base number 5023 in the sequence of SEQ ID NO:1 and a primer composed of the base sequence corresponding to the complementary chain of the 3' side sequence from the base number 5235, and carrying out PCR using these primers and a human genome library as contained in the kit described in Example 2.

### (2) Production using DNA synthesis

The DNA can be also produced by combining several DNA fragments which are divided ones of the sequence and complementary chain thereof corresponding to part of the sequence of SEQ ID NO:1 containing the base sequence from the base number 5023 to the base number 5235 according to a chemical synthetic method and then combining these DNA fragments. Each DNA fragment can be synthesized using a DNA synthesizer (e.g., Oligo 1000M DNA Synthesizer (Beckman), 394 DNA/RNA Synthesizer (Applied Biosystems), or the like).

It can be verified for example by the method described in Example 5 whether the DNA produced has an hPGC-1 promoter activity or not.

For those skilled in the art, it is possible to prepare a DNA having a promoter activity equal to that of natural promoter DNA by modifications such as substitution of part of the base sequence of the promoter sequence present in natural one with other base(s), deletion of the base(s), and/or addition. Thus, a DNA having a base sequence wherein the base(s) is substituted, deleted and/or added in the base sequence of natural one and having a promoter activity equal to that of natural promoter DNA is also included in the DNA's of the invention. The modification of the base(s) can be carried out, for example, by deletion introduction with a restriction enzyme or a DNA exonuclease, mutation introduction according to site-specific mutation induction methods (Nucleic Acid Res. 10, 6487 (1982)), modification of the promoter sequence according to the PCR method, direct introduction of a synthetic mutant DNA, or the like (Maniatis, T. et al. (1989): "molecular Cloning - A Laboratory Manual 2^{nd} Edt." Cold Spring Harbor Laboratory, NY).

It is also possible to utilize the hPGC-1 promoter DNA of the invention for treating or preventing deficiency or expression abnormality of hPGC-1 protein derived from mutation in a living body. For example, hPGC-1 protein can be expressed under a normal transcriptional control by inserting the hPGC-1 promoter DNA of the invention and an hPGC-1 gene containing hPGC-1 coding region into a vector such as retrovirus, adenovirus or adeno-associated virus or by including it in liposome and then by introducing it into somatic cells. Thereby, the improvement of deficiency or expression abnormality of hPGC-1 protein is enabled.

Irrespective of the promoter activity, a DNA containing at least part of the DNA described in SEQ ID NO:1 can competitively inhibit the binding of the hPGC-1 promoter DNA of the invention to a protein capable of binding to it (e.g., transcription factor). Therefore, in the case that the DNA corresponds to the binding site of a protein inhibiting the promoter activity of the hPGC-1 promoter DNA of the invention, the promoter activity can be stimulated by administering the DNA. Contrary, in the case that it corresponds to the binding site of a protein stimulating the promoter activity, the promoter activity can be inhibited by administering the DNA. The DNA for use in the competitive inhibition has a length of usually at least 6 bases or more, preferably 10 bases or more.

The recombinant vector of the invention can be produced by incorporating the DNA of the invention into a vector suitably selected depending on the purpose. For example, in the case of aiming at the construction of a screening system of a substance controlling an hPGC-1 promoter activity, as described in Example, it is preferred to produce the vector by incorporating the DNA of the invention into a vector into which a reporter gene such as luciferase is incorporated. Moreover, in the case of aiming at the gene therapy of deficiency or expression abnormality of hPGC-1 protein, the vector can be produced by incorporating the DNA of the invention and a DNA of hPGC-1 coding region into a vector such as retrovirus, adenovirus or adeno-associated virus.

The transformant of the invention can be produced by incorporating the DNA of the invention into a host cell suitably selected depending on the purpose. For example, in the case of aiming at the construction of a screening system of a substance controlling an hPGC-1 promoter activity, it is preferred to use a cell derived from muscle or adipose tissue derived from a mammal such as human, mouse or rat.

### 2) Screening method of the present invention

A substance stimulating an hPGC-1 promoter activity has an action to activate mitochondrial enzymes in muscle cells (Example 7) and mPGC-1 has an action to induce the expression of decoupling factors causing heat production in mitochondria, increase oxygen consumption, and increase the number of mitochondria in a cell, so that it is considered that a substance stimulating an hPGC-1 promoter activity stimulates the combustion of fat accumulated in cells by enhancing energy metabolism in human cells and as a result causes the reduction of fat tissue mass. Therefore, by screening a substance stimulating an hPGC-1 promoter activity, it seems to be possible to obtain an effective substance for treating or preventing obesity and various diseases derived from obesity, e.g., diabetes (especially non-insulin dependent diabetes mellitus).

The "test substance" to be used in the screening method of the present invention includes a protein, a DNA, and a compound. The compound herein includes a natural product in addition to artificially synthesized one irrespective of a low-molecular-weight molecule or a polymeric molecule, or an organic substance or an inorganic substance. The following will describe a screening method of such a protein, DNA or compound.

It is possible to utilize the hPGC-1 promoter DNA of the invention for screening a substance (protein, DNA or compound) controlling the promoter activity or DNA encoding a protein controlling the promoter activity. The substance controlling the promoter activity includes a substance indirectly controlling the activity by acting on a cell membrane receptor or an intracellular protein in addition to a substance controlling the activity by directly binding to a promoter DNA. The following will describe the screening method of such a substance.

### (1) Screening of a protein controlling promoter activity and a DNA encoding the same

First, a gene library is introduced into a cell (animal cell or yeast) having a reporter gene connected to a downstream of the promoter DNA of the invention and then an expression protein controlling the expression of a reporter gene or a DNA encoding the same is selected. Specifically, as described in Example 5, a vector wherein a reporter gene such as luciferase is connected to a downstream of the promoter DNA of the invention is prepared and a cell expressing it stably or transiently in yeast or an animal cell is prepared. Then, a clone inducing or inhibiting the expression of the reporter gene is selected by introducing a gene library DNA into it and detecting the reporter activity (e.g., luciferase activity), and then a protein or a DNA encoding the same is selected from the clone. By this method, a protein directly binding to the DNA of the invention and controlling the promoter activity or a DNA encoding the same is obtained. Furthermore, it is also possible to obtain a protein indirectly controlling the activity through acting on an intracellular protein, or a DNA encoding the same.

Secondly, a test sample such as a culture supernatant of an animal cell into which a gene library DNA has been introduced is added to a cell containing a reporter gene connected to a downstream of the promoter DNA of the invention, and a clone having a supernatant controlling the expression of the reporter gene is selected. By this method, a protein indirectly controlling the promoter activity via a cell membrane receptor or the like, or a DNA encoding the same can be obtained.

Thirdly, the promoter DNA of the invention is biotinylated and bound to magnetic beads, to which streptoavidin has been bound, to prepare DNA affinity beads, which are then allowed to react with a nuclear extract solution of cells to purify proteins in the nuclear extract solution, which specifically bind to the promoter DNA of the invention. Thereby, a protein directly binding to the DNA or a protein forming a complex with a protein directly binding to the DNA can be obtained.

Fourthly, each protein is expressed in Escherichia coli into which a gene library has been introduced and after the transfer of the protein to a filter membrane, the proteins are directly blotted using the promoter DNA of the invention as a probe, and proteins directly binding to the probe are selected. By this method, a protein directly binding to the DNA or a DNA encoding the same can be obtained.

### (2) Screening of a DNA or a compound controlling the promoter activity

In the case that a test substance is a DNA or a compound, in an almost similar manner as described in (1), for example, a test substance (DNA or compound) is brought into contact with a cell having a reporter gene connected to a downstream of the promoter DNA of the invention and a substance controlling the reporter activity is selected. Specifically, a reporter gene such as luciferase is connected to a downstream of the promoter DNA of the invention, and the gene is expressed stably or transiently in a cell (animal cell or yeast). Then, the cell and a test substance are allowed to react in a culture liquid and a substance inducing or inhibiting the reporter activity is selected. By this method, a substance directly or indirectly controlling promoter activity can be obtained. As a specific screening method, the method described in Example 6 is preferred and as a substance stimulating the promoter activity, it is preferred to select a substance having a promoter activating ability which is twice or more of that in the case of adding no compound.

Alternatively, the promoter DNA of the invention and a protein binding thereto, e.g., a transcription controlling factor or the like are brought into contact with each other in the presence of a test substance and a substance inhibiting or stimulating the binding of the DNA to the protein is selected. Specifically, the promoter DNA of the invention is labeled and the labeled one is then allowed to react, as a probe, with a nuclear extract solution of cells. Then, a polyacrylamide gel electrophoresis is carried out to detect a band of the complex of the probe with a protein in the nuclear extract solution. By adding a test substance at the reaction of the probe with the nuclear extract solution, a substance inhibiting or stimulating the formation of the complex band is selected. By the way, in the case that a protein binding to the promoter DNA has been isolated, a recombinant protein thereof can be also utilized instead of the nuclear extract solution. By this method, a substance directly acting on the binding of the promoter DNA with a binding protein can be obtained. In the case that a protein inhibiting the transcription of hPGC-1 gene is bound to the promoter DNA, it is considered that a substance inhibiting the binding stimulates the transcription of hPGC-1.

In the case that a protein binding to the promoter DNA of the invention has already been obtained, the protein and the promoter DNA of the invention are brought into contact with each other in the presence of a test substance to select a substance inhibiting or stimulating the binding of the protein to the promoter DNA of the invention. Specifically, a protein (which may be a DNA-binding region alone) binding to the promoter DNA of the invention, which has been fused with repeated histidine (histidine tag) or glutathione S-transferase (GST) is purified and bound to a microplate covered with anti-histidine tag antibody or anti-GST antibody, and then biotinylated promoter DNA of the invention is brought into contact with the protein, followed by detecting the binding of the protein to the promoter DNA of the invention with streptoavidinylated alkaline phosphatase. At the addition of the promoter DNA of the invention, a test substance is also added and a substance stimulating or inhibiting the binding of the protein to the promoter DNA of the invention is selected. In the case that a protein inhibiting the transcription of hPGC-1 gene is bound to the promoter DNA of the invention, it is considered that a substance inhibiting the binding stimulates the transcription of hPGC-1.

### 3) Pharmaceutical composition of the present invention

The present invention includes a pharmaceutical composition containing a substance (protein, DNA, or compound) stimulating the promoter activity of the invention, capable of being selected by the screening method described in 2), as an active ingredient. The pharmaceutical composition of the present invention is preferably a pharmaceutical composition for activating mitochondrial or pharmaceutical composition for treating or preventing obesity and/or diabetes based on the mitochondrial activation containing a substance stimulating the promoter activity as an active ingredient.

Examples of the active ingredient include 5-(4-fluorobenzylidene)-2,4-thiazolidinedione, 5- (4-bromobenzylidene)-2,4-thiazolidinedione, 2-(3-aminophenyl)-5-benzoyl-(1H)-benzimidazole and the like which are selected as substances stimulating the promoter activity of the invention. The pharmaceutical composition of the invention is not limited to the pharmaceutical composition containing each of the substances described in Examples as an active ingredient, but it includes all the pharmaceutical compositions for activating mitochondria containing a substance stimulating the promoter activity as an active ingredient and pharmaceutical compositions for treating or preventing obesity and/or diabetes based on the mitochondrial activation. Preferred is a pharmaceutical composition for activating mitochondria.

In this regard, for verifying the action to activate mitochondria or the effect of treating or preventing obesity and/or diabetes, a known method for those skilled in the art or an improved method thereof can be used. For example, the verification of mitochondrial activation can be carried out by the method described in Example 7, and the verification of the effect of treating or preventing obesity and/or diabetes can be carried out by the following method.

### (1) Pharmacological test for verifying an antiobesity effect

After the measurement of the body weight of obesity and diabetes model mice, the mice are randomly divided into groups. A test drug is dissolved or suspended in physiological saline or 0.5% methyl cellulose solution and then administered to the mice orally, subcutaneously, or intraperitoneally at a dose of 0.1 to 300 mg/kg-animal body weight once to three times per day for 7 to 90 days continuously. As a control, physiological saline or 0.5% methyl cellulose solution containing no test drug is administered at the same dose in a similar manner to the case of the test drug. After the administration, the body weight of each mouse is measured and the change of the body weight is determined by subtracting the body weight of each mouse before the administration. In addition, an energy metabolic rate of each mouse is measured. Specifically, a mouse is placed in a closed chamber and partial pressures of oxygen and carbon dioxide in an expiratory air of the mouse are measured (Nature, 406, 415 - 418 (2000)). The energy consumption per unit time is calculated from the resulting oxygen consumption and carbon dioxide formation. Finally, the mouse is dissected and the weight of visceral and subcutaneous fats is measured. The significant difference is tested between each test compound-administered group and the control group, and the difference between the groups is judged as significant in the case that p value is less than 0.05. At that time, the test compound is judged to have an antiobesity effect in any one of the following cases: 1) the change of the body weight is significantly small or reduced in the test compound group, 2) energy consumption per unit time is significantly large in the test compound group, and 3) the weight of visceral fat or subcutaneous fat is significantly small in the test compound group.

### (2) Pharmacological test for verifying an antidiabetes effect

After the measurement of the blood glucose level of obesity and diabetes model mice, the mice are randomly divided into groups. A test drug is dissolved or suspended in physiological saline or 0.5% methyl cellulose solution and then administered to the mice orally, subcutaneously, or intraperitoneally at a dose of 0.1 to 300 mg/kg-animal body weight once to three times per day for 7 to 90 days continuously. As a control, physiological saline or 0.5% methyl cellulose solution containing no test drug is administered at the same dose in a similar manner to the case of the test drug. After the administration, blood is collected from each mouse and the blood glucose level is measured. Moreover, in order to examine an action to improve insulin resistance of the test drug, an oral glucose tolerance test is carried out. Specifically, each mouse is forced to fast overnight and blood is collected (blood glucose level before glucose load is applied). Thereafter, as a glucose load, glucose is compulsively administered orally by means of a stomach sonde at a dose of 2 g/kg-animal body weight. After the administration of glucose, blood is collected with time from each mouse and the difference between the groups is judged as significant in the case that p value is less than 0.05. At that time, the test compound is judged to have an antidiabetes effect in any one of the following cases: 1) the blood glucose level is significantly small in the test compound group, 2) any of the samples at each blood collection time in the glucose tolerance test is significantly small in the test compound group, and 3) AUC (area under the curve) of the change of the blood glucose levels in the glucose tolerance test is significantly small in the test compound group.

The pharmaceutical preparation containing a substance (protein, DNA or compound) stimulating an hPGC-1 promoter activity as an active ingredient can be prepared using carriers, fillers and other additives generally used for the drug formulation depending on the types of the active ingredients.

The administration includes oral administration with tablets, pills, capsules, granules, fine granules, powders, oral solutions or the like, or parenteral administration with intravenous or intramuscular injections, suppositories, percutaneous administering agent, transdermal administering agent or the like.

In the solid composition for oral administration according to the invention, one or more active substances are mixed with at least one inert diluent such as lactose, mannitol, glucose, microcrystalline cellulose, hydroxypropylcellulose, starch, polyvinyl pyrrolidone, magnesium aluminate metasilicate or the like. In the usual way, the composition may also contain other additives than the inert diluent, such as a lubricant, a disintegrating agent, a stabilizing agent and a solubilizing or solubilization assisting agent. If necessary, tablets or pills may be coated with sugar or a film of a gastric or enteric substance.

The liquid composition for oral administration includes emulsions, solutions, suspensions, syrups, and elixirs, and contains a generally used inert diluent such as purified water or ethanol. In addition to the inert diluent, the composition may also contain additives, e.g., a moistening agent, a suspending agent, a sweetener, an aromatic and an antiseptic.

The injections for parenteral administration include aseptic aqueous or non-aqueous solutions, suspensions and emulsions. The aqueous solutions and suspensions contain distilled water for injection and physiological saline as a diluent. Examples of the diluent for the non-aqueous solutions and suspensions include propylene glycol, polyethylene glycol, plant oils such as olive oil, alcohols such as ethanol, polysorbate 80 and the like. Such a composition may further contain additive a moistening agent, an emulsifying agent, a dispersing agent, a stabilizing agent, a solubilizing or solubilization assisting agent, an antiseptic and the like. The composition is sterilized by filtration through a bacteria-retaining filter, blending of a germicide or irradiation. Alternatively, it may be used by firstly making into a sterile solid composition and dissolving it in sterile water or a sterile solvent for injection prior to their use.

The dose is optionally decided by taking into consideration of strength of activity of the active ingredient selected by the above mentioned screening method, symptoms, age, sex and the like of each patient to be administered. For example, in the case of oral administration, the dose is from 0.1 mg to 5,000 mg, preferably from 1 mg to 500 mg, per day for adult (as body weight of 60 kg). In the case of parenteral administration, the dose is from 0.01 mg to 1,000 mg, preferably from 0.01 mg to 100 mg in the form of injections.

### Brief Description of the Drawings

Figure 1 illustrates the structure of luciferase reporter vector containing an upper stream of hPGC-1 gene.
Figure 2 illustrates the activity induction by 9-cis-RETINOIC ACID in various reporter vectors shown in Figure 1. The activity is expressed by a relative value wherein luciferase activity is normalized with β-galactosidase activity and the value in the case of adding no compound is regarded as 1.

### Best Mode for Carrying Out the Invention

The following will explain the present invention in more detail with reference to Examples, but the invention is by no means limited to these Examples. In this connection, unless otherwise stated, the operations can be carried out in accordance with known methods (Maniatis, T. et al. (1989): "Molecular Cloning - A Laboratory Manual 2^{nd} Edt." Cold Spring Harbor Laboratory, NY etc.).

### (Example 1) Culture of cells

Rat myoblast L6 (ATCC, CRL-1458) was maintained Dulbecco's modified Eagle medium containing 10% fetal bovine serum (GIBCO BRL, USA, 11965-092) and cultured in a wet air containing 5% CO₂ at 37°C.

### (Example 2) Cloning of hPGC-1 gene promoter region

An oligonucleotide (SEQ ID NO:2) of 28 bases antisense chain at the 5'-side sequence and an oligonucleotide (SEQ ID NO:3) of 31 bases antisense chain at the 5'-side sequence were synthesized and used as primers for PCR. Using GenomeWalker™ Kit (CLONTECH, USA, K1803-1) for cloning of the promoter region, operations were carried out in accordance with the attached manual. As a result of primary PCR using the DNA described in SEQ ID NO:2 and secondary PCR using the DNA described in SEQ ID NO:3 among the synthetic oligonucleotides, a DNA fragment of about 5.2 kb can be obtained from Sca I library in the kit and a DNA fragment of about 2.2 kb from EcoR V library.

### (Example 3) Sequence analysis of hPGC-1 gene promoters

The DNA fragment obtained in Example 2 was subcloned into pCR®2.1-TOPO vector using TOPO™ TA Cloning® Kit (Invitrogen®, USA, IV450002) in accordance with the attached manual and a subclone of the DNA fragment of about 5.2 kb was named pCR-hPGC1-5' and a subclone of the DNA fragment of about 2.2 kb was named pCR-hPGC1S-5'. The base sequences of the subcloned DNA's were determined using BigDye™ Terminator Cycle Sequencing Kit (ABI PRISM, PE APPLIED Biosystems, USA, 4303125) and ABI PRISM™ 377 DNA Sequencer. The base sequence of the DNA fragment of about 5.2 kb was shown in SEQ ID NO:1. The DNA fragment of about 2.2 kb corresponded to the base sequence of from the base number 2816 to the base number 5235 in the sequence of SEQ ID NO:1.

### (Example 4) Construction of reporter vectors having hPGC-1 gene promoters

Not I-Mlu I fragments containing about 5.2 kb and about 2.2 kb hPGC-1 gene upstream regions were obtained by cleaving pCR-hPGC1-5' and pCR-hPGC1S-5' prepared in Example 3 using restriction enzymes Not I and Mlu I, and the fragments were introduced into Sma I-Mlu I site of PicaGene Basic Vector 2 (Toyo Ink, Japan) to construct hPGC-1 reporter vectors. At that time, each Not cleaved end of the DNA fragments obtained by cleavage is made blunt end using T4 DNA polymerase so as to be able to introduce the fragment into Sma I site. A reporter vector into which the DNA fragment of about 5.2 kb was introduced was named phPGC1(5.2)Luc and a reporter vector into which the DNA fragment of about 2.2 kb was introduced was named phPGC1(2.2)Luc.

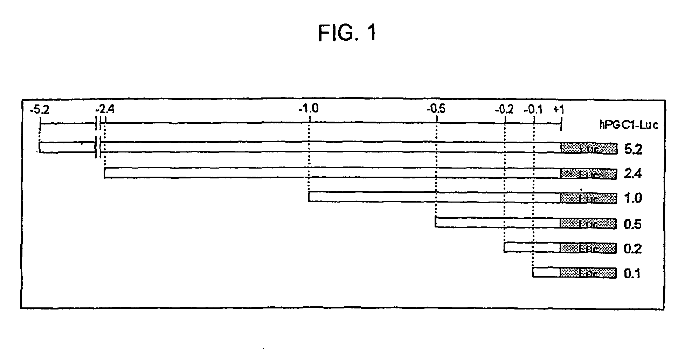

DNA fragments containing hPGC-1 gene upstream regions of about 1 kb (the base sequence of from the base number 4269 to the base number 5235 in the sequence of SEQ ID NO:1), about 0.5 kb (the base sequence of from the base number 4776 to the base number 5235 in the sequence of SEQ ID NO:1), about 0.2 kb (the base sequence of from the base number 5023 to the base number 5235 in the sequence of SEQ ID NO:1), and about 0.1 kb (the base sequence of from the base number 5129 to the base number 5235 in the sequence of SEQ ID NO:1) were obtained by combining the synthetic oligonucleotide shown in SEQ ID NO:3 and a synthetic oligonucleotide shown in SEQ ID NO:4, SEQ ID NO:5, SEQ ID NO:6, or SEQ ID NO:7 as primers. These DNA fragments were introduced into PicaGene Basic Vector 2 and the products were named hPGC1(1.0)Luc, hPGC1(0.5)Luc, hPGC1(0.2)Luc, and hPGC1(0.1)Luc, respectively (Figure 1).

### (Example 5) Measurement of hPGC-1 promoter activity

Various reporter vectors shown in Figure 1 were transiently transfected into L6 cells. Specifically, L6 cells were cultured in a tissue culture dish having a diameter of 10 cm and at the time when it was reached 50% confluent, 5 µg of the above plasmid and 1.5 µg of a plasmid pCH110 containing β-galactosidase gene controlled by β-actin promoter were used in combination in order to standardize gene transfection efficiency and the plasmids were introduced into the cells using FuGENE™6 (BOEHRINGER MANNHEIM, USA, 1814 443). After 24 hours, the cells were transferred into a 96-well culture dish, followed by additional 6 hours of culture. From the reports until now, since it is known that 9-cis-RETINOIC ACID (one of vitamin A acids) increases the expression of PGC-1 gene derived from mouse in cells (Cell, 92, 829 - 839 (1998)), 9-cis-RETINOIC ACID (SIGMA, USA, R4643) having a final concentration of 1 µM was added and the mixture was treated for 24 hours. Then, the cells were dissolved with a cell dissolving liquid LCβ (Toyo Ink, Japan) and luciferase activity thereof was measured using a PicaGene Luminescent Kit (Toyo Ink, Japan, 309-04321). By the way, all the activity measurements were carried out three times per measurement and each experiment was repeated at least twice to confirm the reproducibility.

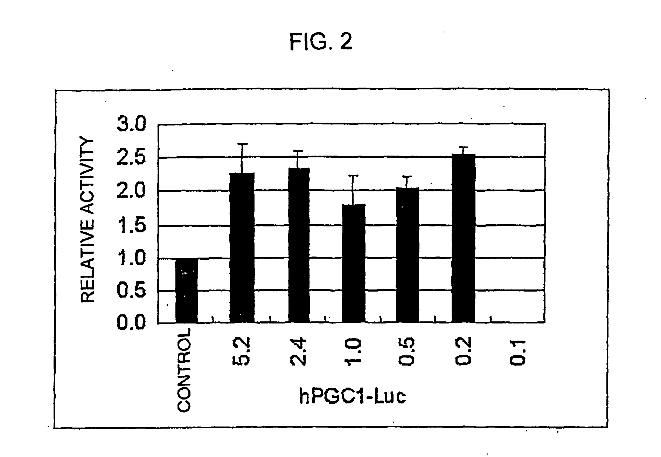

The results are shown in Figure 2. Each reporter activity was standardized by β-galactosidase activity and expressed by a relative value wherein the value in the case of adding no compound was regarded as 1. In L6 cells, hPGC-1 reporter was increased a maximum of about 2.5 times by the treatment with 9-cis-RETINOIC ACID and thus a result consistent with the report that the expression of PGC-1 gene derived from mouse was enhanced by 9-cis-RETINOIC ACID was obtained. Among reporter vectors containing promoters having various lengths prepared, the activity enhancement of 9-cis-RETINOIC ACID responsibility was strongly observed in from hPGC1(0.2)Luc to hPGC1(5.2)Luc. On the other hand, in hPGC1(0.1)Luc, the reporter activity was completely lost, so that it was first shown that the region of from -189 to +23 of hPGC-1 promoter is particularly important for the induction of expression of hPGC-1 gene by 9-cis-RETINOIC ACID.

### (Example 6) Screening of hPGC-1 promoter activating agents

Using the reporter vector phPGC1(2.2)Luc in the method described in Example 5, screening of a compound stimulating an hPGC-1 promoter activity was carried out wherein commercially available compounds were used as test compounds. Under the condition of the compound final concentration of 1 µM, 5 µM or 25 µM, as compounds having a promoter activating ability more than twice of that in the case of adding no compound, Compound A (FAB-MS (M+H)⁺ 324), Compound B (FAB-MS (M+H)⁺ 223), Compound C (FAB-MS (M+H)⁺ 312), Compound D (FAB-MS (M+H)⁺ 374), Compound E (FAB-MS (M+H)⁺ 284), Compound F (FAB-MS (M+H)⁺ 379), Compound G (FAB-MS (M+H)⁺ 313), and the like were obtained, for example. Table 1 shows relative values of each reporter activity (luciferase activity) in the case of adding each compound relative to the reporter activity in the case of adding no compound. By the way, Compound B, Compound E and Compound G are commercially available as the code number 4K@UH1-646 of INTERBIOSCREEN, as the code number MS0447 of MARLIN and as the code number A1773/0075171 of ZELINSKY, respectively and are 5-(4-fluorobenzylidene)-2,4-thiazolidinedione, 5-(4-bromobenzylidene)-2,4-thiazolidinedione, 2-(3-aminophenyl)-5-benzoyl-(1H)-benzimidazole, respectively. Moreover, as a result of structural confirmation by mass spectroscopy and NMR, it was found that Compounds B and E were different from the registered structures and had the structures mentioned above.

**Table 1**

| Compounds | Promoter activity (%) | | |
|---|---|---|---|
| | 1 µM | 5 µM | 25 µM |
| Compound A | 200 | 223 | 144 |
| Compound B | 160 | 203 | 202 |
| Compound C | 195 | 258 | 246 |
| Compound D | 152 | 176 | 202 |
| Compound E | 146 | 208 | 157 |
| Compound F | 155 | 177 | 200 |
| Compound G | 207 | 175 | 91 |

### (Example 7) Measurement of mitochondrial enzymatic activity

For the investigation of mitochondrial enzymatic activity of cells, a reagent WST-1 (DOJINDO, 342-06451) was used, which enabled the measurement of mitochondrial dehydrogenase activity of living cells by colorimetric assay method. Specifically, L6 cells were cultured in a tissue culture dish having a diameter of 10 cm and after reached confluent, the cells were transferred into a 96-well tissue culture dish. At that time, the medium was changed to Dulbecco's modified Eagle medium containing no Phenol Red (GIBCO BRL, USA, 21063-029). After 6 hours of culture, the medium was changed to the medium containing no fetal bovine serum and thereto was added 9-cis-RETINOIC ACID which was found to stimulate hPGC-1 promoter activity in Example 5 in a final concentration of 0.1 µM, 0.3 µM or 1 µM or each compound obtained at the screening in Example 6 in a final concentration of 1 µM, 5 µM or 25 µM. After the addition of each compound and 24 hours of treatment, the medium containing the compound was sucked and discarded and thereto were added 100 µl of Dulbecco's modified Eagle medium containing no Phenol Red and no fetal bovine serum and 10 µL of PBS containing 5 mM WST-1 and 0.2 mM 1-Methoxy PMS (DOJINDO, 345-04001). After 4 hours of reaction at 37°C, absorbance was measured at a measuring wavelength of 450 nm. In this regard, all the activity measurements were carried out three times per measurement and each experiment was repeated at least twice to confirm the reproducibility.

Tables 2(a) and 2(b) show the results of 9-cis-RETINOIC ACID and the compounds obtained in Example 6, respectively. Each value was expressed by a relative value wherein the absorbance of the sample containing no cell was subtracted as a background from each absorbance and the value in the case of adding no compound was regarded as 1. All of 9-cis-RETINOIC ACID and each compound obtained by screening in Example 6 increased mitochondrial enzymatic activity with a significant difference. As mentioned above, it was shown that substances stimulating an hPGC-1 promoter activity have an action to activate mitochondrial enzymes.

**Table 2(a)**

| | | | |
|---|---|---|---|
| Compound concentration (µM) | 0.1 | 0.3 | 1.0 |
| Mitochondrial activity (%) | 1.60*** | 1.70*** | 1.95*** |

| | | | |
|---|---|---|---|
| *** p<0.001 by Dunnett test | | | |

**Table 2(b)**

| Compounds | Mitochondrial activity (%) | | |
|---|---|---|---|
| | 1 µM | 5 µM | 25 µM |
| Compound A | 97 | 140*** | 164*** |
| Compound B | 94 | 121* | 175*** |
| Compound C | 106 | 127*** | 153*** |
| Compound D | 118 | 151** | 198*** |
| Compound E | 102 | 141*** | 285*** |
| Compound F | 115 | 128*** | 140*** |
| Compound G | 80 | 102 | 176*** |

| | | | |
|---|---|---|---|
| p<0.05 ** p<0.01 | | | |
| *** p<0.001 by Dunnett test | | | |

### Industrial Applicability

According to the present invention, a DNA having a promoter activity of hPGC-1 gene was provided, and the screening of a substance controlling the promoter activity is enabled. A substance obtainable through the screening increases mitochondrial activity in muscle cells and realizes the combustion of excessively accumulated fat and the enhancement of energy metabolism by increasing oxygen consumption based on the increased number of intracellular mitochondria and the enhanced function thereof in muscle cells and adipocytes, which cannot be achieved by any known medical agent. As a result, the substance is expected to have an effect of treating or preventing obesity and diabetes.

## Claims

1. A DNA having a human PGC-1 promoter activity, which comprises at least the base sequence of from the base number 5023 to the base number 5235 in the sequence of SEQ ID NO:1 and is a base sequence corresponding to all or part of the sequence of SEQ ID NO:1.

2. The DNA according to claim 1, which is selected from the group consisting of the base sequence of from the base number 5023 to the base number 5235 in the sequence of SEQ ID NO:1, the base sequence of from the base number 4776 to the base number 5235 in the sequence of SEQ ID NO:1, the base sequence of from the base number 4269 to the base number 5235 in the sequence of SEQ ID NO:1, the base sequence of from the base number 2816 to the base number 5235 in the sequence of SEQ ID NO:1, and the base sequence of from the base number 1 to the base number 5235 in the sequence of SEQ ID NO:1.

3. A recombinant vector comprising the DNA according to claim 1.

4. A transformant comprising the DNA according to claim 1.

5. A screening method for a substance controlling the human PGC-1 promoter activity of the DNA according to claim 1, which comprises a step of bringing a test substance into contact with the DNA and a step of measuring the human PGC-1 promoter activity.

6. The screening method according to claim 5, wherein the DNA according to claim 1 has a reporter gene in the downstream and the step of measuring the human PGC-1 promoter activity is a step of detecting the expression of the reporter gene.

7. The screening method according to claim 5, which aims at the selection of a mitochondria-activating substance.

8. The screening method according to claim 7, wherein the mitochondria-activating substance is a substance for treating or preventing obesity and/or diabetes.

9. A substance for controlling a human PGC-1 promoter activity, which is capable of being selected by the method according to claim 5.

10. A pharmaceutical composition for activating mitochondria comprising as an active ingredient a substance stimulating a human PGC-1 promoter activity, which is capable of being selected by the method according to claim 5.

11. The pharmaceutical composition according to claim 10, which is a pharmaceutical composition for treating or preventing obesity and/or diabetes.

12. A process for producing a pharmaceutical composition for activating mitochondria, which comprises a step of screening a substance stimulating a human PGC-1 promoter activity by the method according to claim 5 and a step of preparing a formulation using the substance stimulating the promoter activity.

13. The production process according to claim 12, wherein the pharmaceutical composition for activating mitochondria is a pharmaceutical composition for treating or preventing obesity and/or diabetes.
